# EUROPEAN PATENT SPECIFICATION

(11) **EP 0 850 047 B1**
(45) Date of publication and mention of the grant of the patent: **24.12.2008**
(21) Application number: 96929919.7
(22) Date of filing: 30.08.1996
(51) Int. Cl.: A61K 9/127, A61K 9/133, A61K 31/66, A61K 38/00

(54) **THERAPEUTIC LIPIDIC VESICLES: METHODS OF USE AND COMPOSITIONS**
THERAPEUTISCHE LIPIDVESIKEL, VERFAHREN ZU DEREN VERWENDUNG UND DIESE ENTHALTENDE MITTEL
PROCEDES D'UTILISATION ET COMPOSITIONS DE VESICULES LIPIDIQUES THERAPEUTIQUES

(30) Priority: 31.08.1995 US 521670; 31.08.1995 US 522745
(43) Date of publication of application: 01.07.1998
(73) Proprietor: YISSUM RESEARCH AND DEVELOPMENT CO., 91042 Jerusalem (IL); Kurtz, Seymour, Chicago, IL 60611 (US)
(72) Inventor: KURTZ, Seymour, Chicago, IL 60611 (US); SHMEEDA, Hilary, 90917 Gavat Zev (IL); BARENHOLZ, Yechezkel, 93707 Jerusalem (IL); CHAJEK, Tova, 93707 Jerusalem (IL)
(74) Representative: UEXKÜLL & STOLBERG
(86) International application number: PCT/US1996/014240
(87) International publication number: WO 1997/007785

(56) References cited:
- EP-A- 0 461 559
- WO-A-94/04177
- WO-A-95/34288
- US-A- 4 812 314
- US-A- 5 128 318
- US-A- 5 344 822
- CHEMICAL ABSTRACTS, vol. 100, no. 13, 26 March 1984 Columbus, Ohio, US; abstract no. 98526, REYNOLDS, JAQUELINE A.: "INTERACTION OF APOLIPOPROTEIN A-I FROM HUMAN BLOOD SERUM HIGH-DENSITY LIPOPROTEIN WITH EGG YOLK PHOSPHATIDYCHOLINE" XP002117477 & BIOCHEMISTRY, vol. 23, no. 6, 26 March 1984, pages 1124-1129,
- CHEMICAL ABSTRACTS, vol. 101, no. 19, 5 November 1984 Columbus, Ohio, US; abstract no. 165871, CHACKO, GEORGE K.: "CHARACTERIZATION OF HIGH-DENSITY LIPOPROTEIN IN RAT LIVER AND TESTIS MEMBRANES" XP002117478 & BIOCHIM. BIOPHYS. ACTA, vol. 795, no. 2, 5 November 1984, pages 417-426,
- CHEMICAL ABSTRACTS, vol. 104, no. 15, 14 April 1986 Columbus, Ohio, US; abstract no. 127172, DELAMATRE, J. ET AL.: "ROLE OF APOLIPOPROTEINS IN CELLULAR CHOLESTEROL EFFLUX" XP002117479 & BIOCHIM. BIOPHYS. ACTA, vol. 875, no. 3, 14 April 1986, pages 419-428,
- CHEMICAL ABSTRACTS, vol. 104, no. 15, 14 April 1986 Columbus, Ohio, US; abstract no. 127173, MELZNER, INGO ET AL.: "CHOLESTEROL SYNTHESIS IN CULTURED HUMAN PERIPHERAL LYMPHOCYTES. INFLUENCE OF LDL, HDL, CHOLESTEROL/PHOSPHATIDYLCHOLINE LIPOSOMES AND COMPLETE SERUM" XP002117480 & BIOCHIM. BIOPHYS. ACTA, vol. 875, no. 3, 14 April 1986, pages 439-449,
- CHEMICAL ABSTRACTS, vol. 112, no. 26, 25 June 1990 Columbus, Ohio, US; abstract no. 240366, GINEVRA, F. ET AL.: "DELIVERY OF THE PHOTOSESITIZER ZINC(II)-PHTHALOCYANINE TO SERUM PROTEINS BY DIFFERENT LIPOSOMES: STUDIES IN VITRO AND IN VIVO" XP002117481 & CANCER LETT., vol. 49, no. 1, 25 June 1990, SHANNON, IRELAND, pages 59-65,
- ANNALS OF THE RHEUMATIC DISEASES, 1995, Vol. 54, TAKAHASHI et al., "Increased Concentrations of Serum Lp(a) Lipoprotein in Patients with Primary Gout", pages 90-93.
- METABOLISM, January 1995, Vol. 44, No. 1, YAMAMOTO et al., "Serum Lipoprotein (a) Levels Before and After Subtotal Thyroidectomy in Subjects with Hyperthyroidism", pages 4-7.
- CANCER BIOCHEM. BIOPHYS., 1994, Vol. 14, KOKOGLU et al., "Elevated Serum Lp(a) Levels in the Early and Advanced Stages of Breast Cancer", pages 133-136.
- ORIGINAL ARTICLES, 11 May 1994, GROOP et al., "Lipoprotein (a) in Type 1 Diabetic Patients with Renal Disease", pages 961-967.
- THE AMERICAN JOURNAL OF CARDIOLOGY, 01 June 1994, Vol. 73, No. 15, DAIDA et al., "Prevention of Restenosis After Percutaneous Transluminal Coronary Angioplasty by Reducing Lipoprotein (a) Levels with Low-Density Lipoprotein Apheresis", pages 1037-1040.
- BIOCHIMICA ET BIOPHYSICA ACTA, 1986, Vol. 875, WILLIAMS et al., "Uptake of Endogenous Cholesterol by a Synthetic Lipoprotein", pages 183-194.

## Description

### FIELD OF THE INVENTION

The present invention relates to methods of therapeutic use of a suspension of small unilamellar lipid vesicles and of therapeutic use of a suspension of small unilamellar lipid vesicles associated with A and C apoproteins, which vesicles and apoproteins together form lipoprotein particles. The invention further relates to the composition of the lipoprotein particles and to methods for their formation.

### REFERENCES

Anselem, S., et al., LIPOSOME TECHNOLOGY (Gregoriadis, G., ed), pp. 501-524, CRC Press, Boca Raton, FL (1993).
Bailey, J.M., Exp. Cell Res. 37:175-182 (1965)
Barenholz, Y., et al., Biochemistry 16:2806 (1977).
Barenholz, Y., et al., LIPOSOME TECHNOLOGY (Gregoriadis, G., Ed), pp. 524-607, CRC Press, Boca Raton, FL (1993).
Bisgaier, C.L., et al., Biochim. Biophys. Acta 918:242-249 (1987).
Bisgaier, C.L., et al., J. Biol. Chem. 264 (2):862-866 (1989).
Blankenhorn, D.H., et al., J. Am. Med. Assoc. 257:3233-3240 (1987).
Brissette, L. et al., J. Biol. Chem. 261:11631-11638 (1986).
Chung, B. H., et al., Methods Enzymol. 128:181-209 (1986).
Daida, H., et al., Am. J. Cardiol. 73(15):1037-1040 (1994).
Desmarais, R.L., et al., Circulation 91(5):1403-1409 (1995).
Edelstein, C., et al., J. Biol. Chem. 247:5842-5849 (1972).
Fiske, C. H., et al., J. Biol. Chem. 66:375-400 (1926).
Folch, J., et al., J. Biol Chem. 226:497-509 (1957).
Glomset, J.A., J. Lipid Res. 9:155-167 (1968).
Groop, P.H., et al., Diabet. Med. 11(10):961-967 (1994). Haase, A., et al., Biol Chem Hoppe Seyler, 369:585-593 (1988).
Harrison, T.R., Ed., HARRISON'S PRINCIPLES OF INTERNAL MEDICINE, TWELFTH ED. pp. 839, 1814-1825, McGraw Hill, Inc. (1991).
Hatch, F. T., et al., Adv. Lipid Res. 6:1-68 (1968).
Havel, R.J., et al., J. Clin. Invest. 34:1345-1353 (1955).
Holmquist, L., et al., Biochim. Biophys. Acta 493:400-409 (1977).
Holtfreter, C. et al., Biol Chem Hoppe Seyler 369:1045-1054 (1988).
Kokoglu, E., et al., Cancer Biochem. Biophys., 14(2):133-136 (1994).
Levida, M. Handbook of Nutrition in the Aged (R.R. Watson ed.), CRC Press, pp 89-109 (1985).
Lowry, O. H., et al., J. Biol. Chem. 193:265-275 (1951).
Mallory, J.B., et al., J. Biol. Chem 262:4241-4247 (1987).
Matz, C. E., et al., J Biol Chem 257:4535-4540 (1982).
Miller, G. J., et al., Lancet 1:16-19 (1975).
Newman, H.A.I., et al., J. Lipid Res. 2: 403-411 (1961).
Noel, S.-P., et al., Biochim. Biophys. Acta 754:117-125 (1983).
Rifici, V.A., et al., Biochim. Biophys. Acta 834:205-214 (1985).
Shinitsky, M., et al., J. Biol Chem 249:2652 (1974).
Szoka, F., et al., Ann Rev Biophys Bioeng, 9:467 (1980).
Takahashi, S., et al., Ann. Rheum. Dis. 54(2):90-93 (1995).
Tenda, K., et al., Jpn. Circ. J. 57(8):789-795 (1993).
Yamamoto, K., et al., Metabolism 44(1):4-7 (1995).

The use of unilamellar vesicles composed primarily of phospholipids in the treatment of hypertension, impaired renal function, and atherosclerosis is disclosed in WO 95 342 88 and EP 0 461 559.

### BACKGROUND OF THE INVENTION

Lipoproteins are high molecular weight particles that are primarily responsible for lipid transport, namely of triglycerides and cholesterol in the form of cholesteryl esters, through the plasma. Five major classes of naturally-occurring lipoproteins are known to circulate in plasma, each differing in lipid composition, apoprotein composition, density, size, and electrophoretic mobility. (Harrison, 1991). Each lipoprotein particle is composed of a non-polar core region, a surrounding phospholipid surface coating containing small amounts of cholesterol, and exposed at the surface, apoproteins which are responsible for binding to receptors on cell membranes and directing the lipoprotein carrier to its intended site of metabolism. At least ten different apoprotein molecules have been identified, and each class of lipoprotein particle contains a specific apoprotein (also referred to as apolipoproteins) or combination of apoproteins embedded in its surface (Harrison). These apoproteins are encoded by genes localized to sites on chromosomes 1, 2, 6, 11, and 19, and mutations of these genes are thought to play a role in atherogenesis.

The major classes of lipoproteins found in human plasma include chylomicrons and chylomicron remnant particles, VLDL (very low density lipoprotein), IDL (intermediate density lipoprotein), LDL (low density lipoprotein), and HDL (high density lipoprotein). Chylomicrons contain a hydrophobic core primarily composed of dietary triglycerides and contain several apoproteins including AI, AII, B48, CI, CII, CIII, and E. VLDL contains a core of endogenous triglycerides synthesized in the liver, in addition to apoproteins B48, CI, CII, CIII, and E. IDL particles are composed of lipids including cholesteryl esters and triglycerides and contain apoproteins B100, CIII, and E. A fourth class of lipoprotein, LDL, possesses a core composed almost entirely of cholesteryl esters and has a surface coat containing only apo B100. About three-fourths of the total cholesterol in normal human plasma is contained within LDL particles. A fifth class of lipoprotein, HDL, also contains cholesteryl esters and possesses a surface coating which includes AI and AII apoproteins. A detailed description of the major classes of human lipoproteins and their function in lipid transport is provided in Harrison (Harrison, 1991).

In addition to the major classes of lipoproteins, a lipoprotein-like particle, Lp(a), has been identified and shown to bear a strong resemblance to both lipoprotein and plasminogen. Its protein components include apo B100 linked to apo (a) via a disulfide bridge. Although a relationship has not been clearly established, the structural resemblance of the lipoprotein-like Lp(a) particle to plasminogen is thought to provide a link between lipids, the clotting system, and atherogenesis.

Numerous studies have been undertaken to elucidate the various physiological roles of lipoproteins. The results are often inconclusive, due to the complex nature of lipoproteins and the accompanying complexity of the human lipid transport system. Other findings are derived from in vitro studies or are based on animal models.

In humans, HDL has been shown to be associated with a protective effect against atherosclerosis (Miller, 1975; Blankenhorn, 1987). It has been hypothesized that HDL exerts its protective effect by the reverse transport of excess cholesterol from peripheral tissues to the liver (Bailey, 1965; Glomset, 1968). However, in regard to its role in anti-atherogenesis, the mechanisms of HDL uptake of cholesterol and delivery to the liver remain an issue of debate (Bisgaier, 1988).

In rats, the vast majority of IDL has been shown to be rapidly cleared by the liver. The mechanism of IDL uptake is not clearly understood, although it appears to involve a receptor-mediated process (Noel, 1983).

Other studies have shown various disease associations with above-normal or sub-normal levels of certain lipoproteins. It is known, for example, that serum Lp(a) is elevated in subjects with gout (Takahashi, et al., 1995), various types of cancer, such as breast cancer (Kokoglu, et al., 1994), hyperthyroidism (Yamamoto, et al., 1995), and in Type 1 diabetics with early and established renal disease (Groop, et al., 1994). It has also been shown that there is a direct correlation between development of post-angioplasty restenosis and elevated serum Lp(a) concentrations (Tenda, et al., 1993).

### SUMMARY OF THE INVENTION

In one aspect, the invention relates to a method of reducing the Lp(a) concentration in a subject at risk for developing a disease condition associated with a chronic, elevated Lp(a) concentration, such as gout, breast cancer, hyperthyroidism, coronary heart disease or cerebral vascular disease. The method includes intravenously administering to the subject a suspension of small unilamellar liposomes composed primarily of phosphatidylcholine phospholipids having phase transition temperatures in the range between about -10 and 37°C, preferably a transition temperature of less than about 5°C, as exemplified by egg phosphatidylcholine which has a transition temperature of -5°C.

The liposomes in the composition are small unilamellar vesicles (SUVs), i.e., vesicles having sizes predominantly between 0.02 and 0.12 microns, and preferably 0.02-0.08 microns. The liposome suspension is administered at a dose of between about 50-1,000 mg lipid/kg body weight. Multiple treatments may be given, e.g., at least once a week, over a several week period. Treatment is carried out until a desired reduction in Lp(a) level is observed.

The treatment method may also be applied to a person having an elevated Lp(a) serum concentration and a disease, such as gout, breast cancer or hyperthyroidism, cerebral vascular disease, and coronary heart disease associated with such elevated concentration.

In another aspect, the invention relates to a method of inhibiting restenosis in a subject, following percutaneous transluminal coronary angioplasty or surgical resection of vascular tissue. The method includes administering liposomes, as described above, until a reduction in serum Lp(a) concentration is observed.

In another aspect, the invention relates to a method of improving the periodontal condition in a person having symptoms of periodontal disease, such as gingivitis, tooth mobility or bone loss. In this method, a suspension of liposomes of the type described above is administered until a significant improvement in the condition is observed. The invention further embodies the first medical use of the described liposome suspensions for the foregoing conditions.

Small unilamellar vesicles may also associate with apoproteins, forming lipoprotein particles. Thus, another aspect of the invention is a lipoprotein composition which contains lipoprotein particles. These particles are composed of small unilamellar vesicles of phosphatidylcholine phospholipids and associated apoproteins selected from apoprotein classes A and C. The phosphatidylcholine phospholipids have phase transition temperatures between about -10 and 37°C and are preferably egg phosphatidylcholine phospholipids. The particles form a therapeutic composition for administration to a subject by intravenous administration.

The particles and composition are formed, in one embodiment, by mixing SUVs of phosphatidylcholine phospholipids with a mixture of apoproteins consisting essentially of apoproteins from classes A and C, typically apo A-1 and C class lipoproteins. The components are mixed under conditions effective to form lipoprotein particles in which the apoproteins are vesicle-associated. Typically, the vesicles contained in the composition have sizes between 0.03 and 0.08 microns.

In one general embodiment, the mixing is carried out *ex vivo,* e.g., by mixing SUVs with a mixture of recombinant A and C apoproteins. In another general embodiment, the mixing is carried out in *vivo* by administering the SUVs to a human subject, allowing the liposomes to circulate for a period of at least about 2 hours, and then optionally, isolating a serum sample from the subject, and isolating from the serum sample. The lipoprotein particles have a density of between about 1.0006 and 1.019 g/ml.

The particle composition is used, in accordance with another aspect of the invention, for treating a disease state which is responsive to intravenous administration of small unilamellar phosphatidylcholine vesicles, in the dosage range preferably between about 1-50 mg vesicle lipid/kg subject body weight. The treatment method includes administering to a subject in need of such treatment, a therapeutically effective dose of a lipoprotein composition, and repeating the administering, if necessary, until a measurable improvement in the disease state is observed.

Another aspect of the invention relates to a method of treating a disease state which is associated with elevated levels of Lp(a), by administering to a subject having an elevated serum Lp(a) level, a therapeutically effective dose of the lipoprotein composition, with repeated administration, if necessary, until a measurable reduction in serum Lp(a) level is observed.

In another aspect, the method is used in treating acute renal failure in a subject, as evidenced by a ratio of urine-to-plasma creatinine less than about 20, where the composition is administered until a significant increase in urine-to serum creatinine ratio is achieved.

In another aspect, the method is used in treating hypertension in a subject having elevated diastolic blood pressure, where the composition is administered until a significant reduction in blood pressure, e.g., a 20% reduction in diastolic blood pressure, is achieved. The invention further embodies the first medical use of the described lipoprotein composition for the foregoing conditions.

These and other objects and features of the invention will become more fully apparent when the following detailed description is read in conjunction with the accompanying figures and examples.

### BRIEF DESCRIPTION OF THE FIGURES

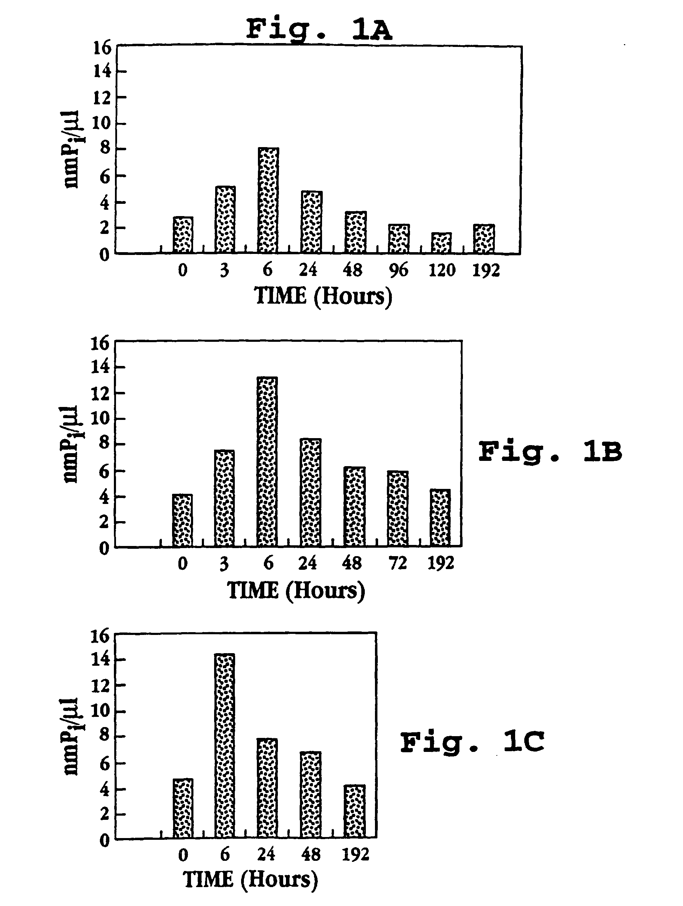

Figs. 1A-1C are bar graphs showing serum phospholipid phosphorus levels in three human subjects (Fig. 1A, Fig. 1B, Fig. 1C, respectively) for a period of 8 days, where continuous liposome infusion occurs in hours 0-6.

### DETAILED DESCRIPTION OF THE INVENTION

### I. Definitions

Unless indicated otherwise, the terms below have the following meanings:

"Small unilamellar vesicles" (SUVs) refer to small single-bilayer liposomes having sizes between about 0.02 to 0.12 microns, preferably 0.02 to 0.08 microns.

"Empty" liposomes refers to liposomes that do not contain entrapped or encapsulated drug.

"Associated apoproteins" in the context of the present invention refers to apoproteins which are stably bound to the SUVs, as evidenced by their ability of SUVs and associated apoproteins to co-migrate in density centrifugation.

"Recombinant apoprotein" refers to an apoprotein which is prepared by genetic engineering techniques commonly known in the art. Recombinant apoproteins are produced typically by cloning the gene of interest into a vector for introduction into any of a number of expression systems such as *E*. *coli,* yeast, cultured mammalian cells, and the like.

A "significant improvement" in a disease state is a measurable degree of improvement, as indicated by either a clinical or biochemical indicator, in the disease state. Typically, a significant improvement in a disease state is one which results in an improvement of a parameter with a known correlation to the disease state of at least five percent. For example, with respect to conditions associated with elevated serum LP(a) levels:
"Elevated Lp(a) concentration" refers to a serum Lp(a) concentration above 25 mg/dl.
"Chronic elevated Lp(a) concentration" refers to a concentration of Lp(a) that is on average elevated above normal average serum Lp(a) concentrations when measured at various times over the course of a week. A normal average serum Lp(a) concentration is generally below 25 mg/dl.
"Significant reduction in Lp(a) concentration" refers to a reduction of at least 20%, preferably more than 40%, with respect to the pretreatment Lp(a) concentration in a subject.
"Significant reduction in blood pressure" refers to a reduction of at least about 10% in diastolic pressure.

### II. Preparation of Lipidic Vesicles

One aspect of the invention relates to therapeutic use of small unilamellar vesicles. This involves administering to a human subject a suspension of liposomes to reduce serum Lp(a) concentrations. The subject in need of such treatment is one having or at risk of developing a disease condition, such as those mentioned above, associated with a chronic, elevated Lp(a) concentration. In another aspect, the invention involves therapeutic use of a lipoprotein suspension, and its composition. The lipoprotein suspension is used in intravenous administration to a subject. The lipoprotein composition contains lipoprotein particles composed of SUVs having associated apoproteins selected from the apoprotein classes A and C. Preparation and use of the lipidic vesicles of the present invention is described in the Examples and in the sections which follow.

### A. Composition of Liposomes

In one embodiment, the liposomes are composed predominantly (more than 50 mole percent, preferably more than 80-90 mole percent) of phosphatidylcholine (PC) having a phase transition temperature less than about 37°C, preferably between about -10 to 24°C, e.g., about 5°C or less.

The lipid composition used in the method of the present invention is composed primarily of PC phospholipids. PC phospholipids include those phospholipids having a choline moiety and two fatty acid chains, each of which may vary in length and in degree of unsaturation.

One preferred vesicle composition includes egg PC, which has a transition temperature of -5°C, and contains predominantly 1-palmitoyl, 2-oleyl PC and 1-palmitoyl,2-linoleyl PC. Alternately, PC may be isolated from rat liver (Newman, 1961), followed by purification on alumina (Shinitzky, 1974).

The liposomes may be composed entirely of egg PC, or may contain other lipid components which (i) are not immunogenic, and (ii) do not contribute a significant portion, i.e., more than 25-50 mole percent, of lipids with high phase transition temperature.

When lipid vesicles alone are used therapeutically, the collective phase transition temperature of vesicles comprising lipids from different phospholipid classes is preferably less than 5 C.

Additional liposome components may include negatively charged lipids, such as phosphatidylglycerol (PG) or phosphatidylserine (PS). Of course, the mole percentage of these lipids should be relatively low with respect to PC. The liposomes may also include cholesterol or other sterols, in an amount preferably less than about 40 mole percent.

Lipid protective agents, such as α-tocopherol, α-tocopherol acetate, or α-tocopherol succinate, may also be included in the lipids forming the liposomes, to protect the lipid components against free radical damage (Levida 1985). Typically such agents are included at a mole percentage between about 0.5% and 2%. It is advantageous to add α-tocopherol to the liposomes to maintain a balance between vitamin E and polyunsaturated lipids in the liposomes.

### B. Preparation of Unsized Liposomes

A variety of methods for producing liposomes are available, and these have been extensively reviewed (Szoka et. al., 1980). In general these methods produce liposomes with heterogeneous sizes from about 0.02 to 10 microns or greater. As will be discussed below, liposomes which are relatively small and well-defined in size are preferred for use in the present invention, hence a second processing step for reducing the size and size heterogeneity of liposomal suspensions will usually be required.

In one preferred method for forming the initial liposome suspension, the vesicle-forming lipids are taken up in a suitable organic solvent system, preferably in a siliconized glass vessel, and dried in *vacuo* or under an inert gas to form a lipid film. An aqueous suspension medium, such as a sterile saline solution, is added to the film, and the vessel is agitated (e.g., on a shaker or using a sonicator) until the lipids have hydrated to completion, typically within about 1-2 hours. The amount of aqueous medium added is sufficient to produce a final liposome suspension containing preferably between about 10 and 30 g lipid per 100 ml media.

During the hydration stage, the lipids hydrate to form multilamellar vesicles (MLVs) with sizes ranging from about 0.5 microns to about 10 microns or larger. In general, the size distribution of MLVs can be shifted toward slightly smaller sizes by hydrating the lipids under more vigorous agitation conditions.

The preparation of egg PC MLVs, followed by treatment of the MLVs with ultrasonic irradiation to reduce the liposome sizes to produce the desired SUVs, is described in the Examples. Sizing methods for producing SUVs from larger multilamellar vesicles are described in further detail below. The aqueous medium used in forming the liposomes may contain water-soluble agent(s) which enhance the stability of the liposomes upon storage. A preferred stabilizing agent is an iron-specific trihydroxamine chelating agent, such as desferrioxamine. The use of this compound in reducing lipid peroxidation and free radical damage in drug-containing liposomes has been reported in U.S. Patent No. 4,797,285. Briefly, it was shown that the combination of a lipophilic free-radical quencher, such as α-tocopherol, and the water-soluble chelator gave substantially better protection against lipid peroxidation damage than did either of the protective agents alone. The chelator is included in the aqueous medium in molar excess of the amount of free iron in the medium. Typically, a chelator concentration of between about 10-200 micromolar is sufficient for reducing lipid peroxidation and free radical damage.

### C. sizing Liposomes

The suspension of liposomes prepared as described above is preferably further treated to produce liposomes having a desired size and size homogeneity. The liposome suspension is generally sized to achieve a selective size distribution of vesicles in a size range less than about 1 micron and preferably less than about 0.8 microns. Liposomes in this size range can be readily sterilized by filtration through a depth filter. Smaller vesicles also show less tendency to aggregate on storage, thus reducing the potential for serious vascular blockage problems upon intravenous administration of the final liposome composition of the present invention. Finally, liposomes which have been sized down to the submicron range possess more uniform biodistribution and drug clearance characteristics.

Preferred liposomes are small unilamellar vesicles (SUVs), *i.e.,* single-bilayer liposomes having sizes between about 0.02 to 0.08 microns. SUVs have been shown to possess relatively long blood circulation halflives, when administered intravenously, as described in co-owned U.S. patent application Serial No. 08/257,899, filed on June 10, 1994. Briefly, as described therein, plots of liposome retention in the bloodstream, measured up to 1,000 minutes after IV injection, revealed that significant quantities of liposomes remained in the bloodstream even at 1,000 minutes.

Several techniques are available for reducing the sizes and size heterogeneity of liposomes, in a manner suitable for preparing the SUVs of the present invention. Ultrasonic irradiation of a liposome suspension either by bath or probe sonication produces a progressive size reduction down to SUVs. A sonicating procedure used to produce SUVs is described in the Examples.

Homogenization is another method which relies on shearing energy to fragment large liposomes into smaller ones. In a typical homogenization procedure, MLVs are recirculated through a standard emulsion homogenizer until selected liposome sizes, typically less than 0.1 microns, are observed.

Extrusion of liposomes through a small-pore polycarbonate membrane is an effective method of reducing liposome size down to a relatively well-defined size distribution. An average range is between about 0.03 and 1 micron, depending on the pore size of the membrane, such as described in the Examples. Typically, the suspension is cycled through the membrane several times until the desired liposome size distribution is achieved. The liposomes may be extruded through successively smaller pore membranes, to achieve a gradual reduction in liposome size. Liposome particle sizes can be determined by a number of techniques including electron microscopy, comparative chromatography (Bisgaier, 1989) and quasi-elastic light scattering.

The size-processed liposome suspension may be readily sterilized by passage through a sterilizing membrane having a particle discrimination size of about 0.2 microns, such as a conventional 0.22 micron depth membrane filter. If desired, the liposome suspension can be lyophilized for storage and reconstituted shortly before use.

### D. Apoprotein Components

The lipoprotein composition of the present invention contains lipoprotein particles composed of SUVs as described above, and molecules of serum proteins, namely human apoproteins, associated with the SUVs. Based upon the applicants' identification of a new lipoprotein fraction isolated from blood samples withdrawn from subjects post-SUV infusion, preferred apoproteins are class A and C apoproteins, and particularly apo A-1 and apo Cs.

Apoproteins, a component of naturally-occurring lipoproteins, may be recovered from samples of the corresponding lipoprotein containing such apoprotein(s), typically derived from either human or rat. In an exemplary procedure, the desired lipoprotein fraction is first isolated from plasma by ultracentrifugation (Hatch, 1968; Chung, 1986) using an appropriate density gradient, followed by purification, typically dialysis.

Typical sources for the A and C apoproteins are HDL and VLDL, although any lipoprotein containing A and C apoproteins is suitable. Generally, high density lipoproteins are isolated by ultracentrifugation at densities from 1.063-1.21 g/ml (Havel, 1955), followed by washing by reflotation at a density of 1.21 g/ml.

Isolation of HDL₃, one potential source of apo A-1, has also been described (Brissette, 1986). Briefly, human HDL₃ is prepared by sequential ultracentrifugations between densities 1.125 and 1.21 g/ml and isolated at density 1.21 g/ml (4.6 x 10⁶ g-h). The HDL₃ is then washed under similar conditions, followed by dialysis against a saline solution containing EDTA and sodium azide.

VLDL, from either rat serum or human plasma, is similarly isolated using ultracentrifugation with an appropriate density gradient. In a typical procedure, VLDL is isolated from human plasma by ultracentrifugation at 8.5 x 10⁴ g for about 0.5 h to remove chylomicrons, followed by two rounds of ultracentrifugation at 1.8 x 10⁶ g-h to isolate and wash the VLDL (Brissette, 1986).

Isolation of apoproteins from the corresponding lipoprotein fraction can be carried out by a number of suitable techniques. Both apo A-I and A-IV can be recovered from HDL. In one such method, apo A-1 and A-IV are isolated from HDL by gel filtration and ion-exchange chromatography (Edelstein, 1972; Rifici, 1985). Apo A-1 may also be extracted from a solution of human HDL₃ by precipitation with acetone, followed by centrifugation (Brissette, 1986). In a similar fashion, apo E and apo Cs can be isolated from VLDL using selective extraction procedures (Holmquist, 1977).

Generally, isolated apoprotein fractions are identified by either immunodiffusion analysis with antibodies against the target apoprotein(s) (Bisgaier, 1987) or by polyacrylamide gel electrophoresis. A suitable gel system for carrying out electrophoretic analysis is 4-18% polyacrylamide gradient gel in the presence of sodium dodecyl sulfate and β-mercaptoethanol. A typical polyacrylamide gel electrophoretic mobility pattern for a mixture containing apo E, apo A-I, apo A-II, apo Cs and apo Bs reveals a progression, taken from the origin, as follows: apo Cs and A-II, apo A-I, apo E, apo B₁ and apo Bₕ, as provided in Brissette (Brissette, 1986). In some instances, the isolated apolipoproteins may optionally contain a radiolabel such as iodine-125. Radiolabelled apoproteins may be useful in initial experiments for forming the lipoprotein particles of the present invention by providing a means for estimating the level of apoprotein-SUV association.

Alternatively, apoproteins for use in the present invention may be formed recombinantly. Expression of apoprotein genes is known in the art. Human apo C-II can be prepared by cloning a full length human apo C-II cDNA insert into the pSP19 expression vector, followed by transcription and translation *in vitro* (Holtfreter, 1988). Mature apo CII can be expressed in E. coli transformed with the pKK233-2 apo CII clone, or alternatively, can be formed on a preparative scale by integrating apo CII CDNA into the pUR291 vector (Holtfreter, 1988).

Expression of human apo A-I and apo A-II genes in Xenopus laevis ooctyes has also been described (Haase, 1988). In following the method of Haase, apo A-I and apo A-II genes can be isolated as clones and transcribed and translated in Xenopus laevis oocytes. Simultaneous injection of the apo A-I and apo A-II genes leads to secretion of both apoproteins, which may then be separated by density gradient centrifugation as has been described. Apo A-1 can also be produced in Chinese hamster ovary (CHO) cells by transfection of the CHO cells with an expression plasmid which places the human apo A-I gene under the direction of the human metallothionein II gene promoter (Mallory, 1987).

### E. Forming the Lipoprotein Composition

### IN VITRO METHOD:

The therapeutic lipoprotein composition provided by the present invention contains lipoprotein particles of the invention composed of small unilamellar vesicles of phosphatidylcholine phospholipids having associated A and C apoproteins. The lipoprotein particles of the present invention may be prepared by both *in-vitro* and *in-vivo* methods, as will herein be described.

In accordance with one method of the invention, the lipoprotein particles are formed by mixing phosphatidylcholine (PC) SUVs with apoproteins under conditions suitable for forming apoprotein-associated SUVs. In one such exemplary approach, PC liposomes prepared as described above are incubated with apoprotein. Typically, apoprotein is incubated with the SUVs for a period of at least 1 hour and preferably for 4-8 hours, at temperatures between about 25°C-37°C. To determine the appropriate concentration of apoprotein, the incubation is typically performed at several different concentrations of protein and the degree of associated is determined, based upon the amount of free apoprotein remaining. For each apoprotein contained in the mixture, the protein/phospholipid ratio will typically range from about 0.1-1 (on a µg-to µg basis), with concentrations of SUVs and protein contained in the mixture between about 10-80 µg/ml.

Apoproteins for use in forming the lipoprotein particles of the present invention include apo As (primarily apo A-1, apo A-II, apo A-IV) and apo Cs (primarily apo C-I, apo C-II, apo C-III). In one embodiment, the lipoprotein particles contain apo A-I and apo Cs. The apoproteins may be derived from extracted lipoprotein sources or may be recombinant apoproteins, as described above.

After formation of the lipoprotein particles, excess or non-associated apoprotein is removed from the mixture, typically by washing followed by centrifugation. Following verification of purity, the resulting suspension of lipoprotein particles may be stored as a suspension, or alternatively, the lipoprotein particles may be isolated.

Isolation of the newly formed lipoprotein particles is carried out using a Ficoll gradient flotation protocol. In one such exemplary isolation method, the lipoprotein particles are mixed with a Ficoll solution in phosphate-buffered saline and placed in a centrifuge tube. The solution is then overlayed with Ficoll, followed by a layer of phosphate-buffered saline. The resulting mixture is then centrifuged. Lipoprotein particles floated to the saline and Ficoll layers are collected and the procedure is repeated. The recovered liposomes may be analyzed by SDS-PAGE, typically carried out on acrylamide gel under reducing conditions. Based upon analysis of the resulting electrophoretic patterns, the lipoprotein particles of the present invention are identified by a distinct band having an electrophoretic mobility and density distinct from those of the reaction components and from naturally-occurring lipoproteins. The lipoprotein particles of the present invention are characterized by densities between those of VLDL and LDL, namely between 1.0006 and 1.019 g/ml. The ratio of protein/phospholipid (m/m) in the isolated lipoprotein fraction will generally be between about 0.05-0.5 for each of the apoproteins present.

Protein levels in the recovered lipoprotein particles can be verified using the method of Lowry (Lowry, 1951). Phospholipid concentrations can be determined as described in Example 5 or by digestion with perchloric acid to promote phosphate cleavage (Fiske, 1926).

In an alternate embodiment, the lipoprotein particles are formed by incorporating apoprotein directly during SUV preparation. In this approach, SUVs are prepared and sized as described in Examples 1 and 2, with the exception that apoprotein is included with the vesicle-forming lipids used to form the initial lipid film. Protein is added at a protein/phospholipid (mass/mass) ratio typically between about 0.1-1.

Alternately, the cholate-lipid dispersion method can be used to complex purified A and C apoproteins to egg PC liposomes containing cholesterol (Matz, 1982).

### IN VIVO METHOD:

In accordance with another aspect of the present invention, the lipoprotein particles of the invention are formed by intravenously administering small unilamellar vesicles to a subject, allowing the liposomes to circulate in the bloodstream, and optionally isolating from a blood sample withdrawn from the subject, the lipoprotein particles of the invention.

Preparation of lipoprotein particles according to this aspect of the invention is supported in Examples 3 and 6, and will be further described below.

In this approach, small unilamellar vesicles composed of phosphatidylcholine, as described in Examples 1 and 2 and sections IIA-C herein, are intravenously administered to an animal or human subject. The vesicles may be administered in a single dose, or in multiple doses. The amount of liposomes administered at each dose is between about 10 and 1000 mg lipid per kg of body weight, and preferably between about 50-1000 mg lipid per kg of body weight. After allowing the injected liposomes to circulate in the bloodstream for a period of several hours, preferably at least 2 hours, e.g., 6-24 hours, a blood sample is removed from the subject. The lipoprotein particles of the present invention may be isolated from serum and used as described above. The lipoprotein particles form in the bloodstream.

The post-infusion lipoprotein particles are typically separated from naturally occurring lipoproteins by ultracentrifugation, the method of which has been previously described herein. The lipoprotein particles are purified by gel electrophoresis, typically using a gradient of polyacrylamide. The lipoprotein particles of the invention, as formed by intravenous administration of PC SUVS, are characterized by a density between that of VLDL and LDL, namely between about 1.0006 and 1.019 g/ml.

### III. Therapeutic Uses of Liposome SUVs

This section describes various treatment methods which involve intravenous administration of liposome SUVs of the type described above. In these methods, liposomes are preferably infused two or more times during a period of at least two weeks, and at a dosing frequency of at least one time per week. A preferred dosing frequency is one-two times per week, although single-dose treatment is also contemplated. The dosing periods, e.g., two weeks, may be interrupted by a wash-out period, typically of 1-4 weeks. The treatment, e.g., involving repeating dosing and wash-out periods, may continue over an extended period of several months or more.

The amount of liposomes administered at each dose is between about 10-1,000 mg lipid per kg of body weight, and preferably between about 50-1,000 mg lipid per kg of body weight, although the dose may be substantially less. Long term dosages are typically delivered at a rate of between about 0.001-1 g lipid per kg body weight per day. In a preferred embodiment, the liposome suspension is administered one time per week, at a dose of about 200-500 mg lipid/kg body weight.

A typical dose for an 80 kg individual would be between about 10 and 80 grams lipid, corresponding to between 100 and 800 ml of 10% liposome (w/w) suspension. Administration may be by iv (intravenous) injection, but is preferably done by iv drip (infusion) over a period of at least about 1-2 hour, to minimize discomfort at the site of administration. The liposomes may be suspended in sterile saline or in a nutritional or drug-containing buffer or medium, such as a glucose/salt medium, to combine liposome treatment with other parenteral therapy.

These methods rely on the presence of administered liposomes in the bloodstream over an extended period following iv administration. Liposome levels in the blood following iv administration can be monitored by measuring serum levels of phospholipids, e.g., phosphorus. Figs. 1A-1C show phospholipid phosphorus levels measured in three human subjects following a 6 hour iv infusion of liposomes. Liposomes, prepared as described in Example 1, were administered to each subject (Fig. 1A, 1B, and 1C, respectively) and serum phospholipid phosphorus and free cholesterol levels were monitored. Phospholipid phosphorus levels were measured prior to liposome infusion (time=0) to establish a base level in each patient. The liposomes were infused continuously over a period of 6 hours and blood samples were taken midway through the infusion period (time=3 hours) and at the end of infusion (time=6 hours). Post-infusion, each subject was monitored for 192 hours (8 days) and blood samples were taken at various times.

As seen, the concentration of liposomes in the bloodstream is greatest at the end of the infusion period (time=6 hours), and phosphorus levels greater than normal levels were observed for at least 48 hours post infusion, indicating the presence of exogenous circulating phospholipids. The results indicate that with a liposome infusion time of 6 hours, liposome dosing every two days would be effective in maintaining elevated liposome levels during the dosing period. However, it is noted that the dosing schedule can be more infrequent, since it is not necessary to maintain elevated liposome levels continuously in the treatment method.

### A. Reducing the Disease Risk Associated with Elevated Lp(a)

One aspect of the invention relates to a method of reducing the serum Lp(a) concentration in a person at risk for developing a disease condition associated with a chronic elevated serum Lp(a) concentration. Conditions associated with elevated Lp(a) concentrations include, for example, gout, breast cancer and hyperthyroidism. Coronary heart disease and cerebral vascular disease are other conditions associated with high Lp(a) levels.

A chronically elevated Lp(a) concentration refers to a serum Lp(a) concentration that is above about 25 mg/dl, typically representing an average of Lp(a) values, when measured several times over the course of a week. Serum Lp(a) concentrations can be measured by a variety of methods, including enzyme-linked immunoabsorbent assay (ELISA), latex immunoassay or immunoradiometric assay. A specific kit for determining Lp(a) concentration in a blood sample, Macra^{™}, is available from Terumo Diagnostics (Elkin, MD).

The treatment method, as applied to three male subjects, is described in Example 3. Briefly, each subject received a series of intravenous infusions of liposomes prepared as described in Example 1, and at the dose schedule described in Example 3. At times just before liposome infusion (0 hours) and 24 hours after liposome infusion (24 hours), Lp(a) concentrations were measured, with the results shown in Table 1. Although several time points were not measured, it is clear from the data that a significant reduction in Lp(a) was observed in all cases. For example, patient two, who had a relatively normal serum Lp(a) showed a drop from an initial concentration of 18 mg/dl to a concentration of 14 mg/dl at the end of the treatment period, i.e., a 29% drop in serum Lp(a) concentration. Subject 3 had an initial serum Lp(a) concentration of 32 mg/dl, a concentration in the elevated Lp(a) range. At the end of the treatment period, the Lp(a) concentration was reduced to 18 mg/dl, a 77% reduction.

**Table 1**

| **Injection No.** | **Dose (mg/kg)** | **Time (hours)** | **Lp(a) (mg/dl)** | | |
|---|---|---|---|---|---|
| | | | **Subject 1** | **Subject 2** | **Subject 3** |
| 1 | 200 | 0 | - | 18 | 32 |
| | | 24 | - | - | 27 |
| 2 | 300 | 0 | - | - | 20 |
| | | 24 | - | - | 18 |
| 3 | 300 | 0 | - | 3.8 | - |
| | | 24 | - | 2.6 | 15 |
| 4 | 300 | 0 | - | 3 | 11 |
| | | 24 | - | 3 | 9 |
| 5 | 300 | 0 | 37 | 16 | 24 |
| | | 24 | - | 15 | - |
| 6 | 300 | 0 | - | 14 | 17 |
| | | 24 | - | - | 10 |
| 7 | 300 | 0 | 25.6 | - | - |
| | | 24 | 18.8 | - | 18 |

These results demonstrate the ability of this method to produce a significant reduction in serum Lp(a) levels.

### B. Treatment of Diseases Associated with Elevated Lp(a)

Another aspect of the invention relates to a method of treating gout, breast cancer, or hyperthyroidism in a subject having one of these conditions and an elevated serum Lp(a) concentration. Studies have shown that serum Lp(a) concentrations are elevated in many subjects with gout (Takahashi, *et al.*, 1995), various types of cancer, such as breast cancer (Kokoglu, et al., 1994), and hyperthyroidism (Yamamoto, *et al*., 1995). The purpose of this method is to treat the clinical disease by lowering Lp(a) levels, as one of the underlying factors contributing to the disease.

Treatment in accordance with this method involves first determining serum Lp(a) concentration in a person having one of the above clinical conditions. A patient having an elevated Lp(a) level is then selected as a candidate for the liposome infusion method, as described above, typically as an adjuvant to another treatment method, such as surgery, chemotherapy, or radiation therapy in the case of breast cancer. Treatment is maintained until a significant reduction in Lp(a) is observed and preferably throughout the treatment period for the clinical disease.

### C. Treatment of Restenosis

Restenosis occurs in approximately 20-30 percent of patients following percutaneous transluminal coronary angioplasty. Restenosis can also occur in patients following surgical resectioning of vascular tissue. In this procedure, a region of stenosis in a vessel is removed and the vessel is sutured closed. Restenosis in each case is apparently the result of excessive local myointimal hyperplasia, brought about by platelet aggregation to the freshly dilated or sutured vessel surface (Harrison, 1991).

Recent studies have shown that high serum Lp(a) concentrations are associated with an increased incidence of restenosis after balloon angioplasty (Daida, *et al.*, 1994; Desmarais, *et al.*, 1995; Tenda, *et al.*, 1993). In one study, patients with a serum Lp(a) level of 38 mg/dl had a significantly higher level of restenosis than patients with a serum level of 19.9 mg/dl (Tenda, *et al.*, 1993).

The present invention relates to a method a reducing the extent of restenosis following procedures such as balloon angioplasty or surgical resectioning of vascular tissue. Typically in the method, a person undergoing such a procedure that can lead to restenosis is given one or more pretreatment infusions of liposomes, particularly where existing Lp(a) levels are elevated, to achieve a reduction in such levels. Following the procedure, the patient is again monitored for Lp(a) serum concentrations, and given further liposome infusions if necessary to maintain or achieve low Lp(a) levels, e.g., 20 mg/dl or lower. The treatment may be discontinued after a period of several weeks or more when the risk of restenosis has passed.

### D. Treatment of Periodontal Disease

The invention also relates to a method of improving the periodontal condition of a person suffering from periodontal disease, as evidenced, for example, by gingivitis, bone resorption, pocket formation or tooth mobility. Liposomes were administered, in accordance with this method of the invention, to a male subject having a history of periodontal disease, as evidenced by severe bleeding gingiva, Class II and III mobility and pocket depths between 4-8 mm. Liposomes were infused according to the dosing schedule described in Example 3, and following treatment a significant improvement in the patient's dental health was observed. Periodontal pocket depths decreased to between 2-4 mm, except around two teeth where the pocket depth remained at 8 mm. Periodontal scaling and root planing indicated that very little bleeding was present, and teeth mobility was reduced to Class I. Treatment by liposome administration may be continued until a desired improvement in gum condition is achieved.

### E. Treatment of Baldness (not according to the invention)

In another aspect, a method of improving hair regrowth in a person having alopecia or male pattern baldness is also described. An elderly male subject with scalp hair loss was treated with liposomes, administered according to the dosing schedule described in Example 3. The subject noticed a significant improvement in hair regrowth after liposome treatment.

### IV. Therapeutic Uses of Lipoprotein Particle Compositions

This section describes treatment methods which involve intravenous administration of the lipoprotein particle composition described above. In all of these methods, the composition is administered intravenously at a dose and dosing frequency effective to produce a desired improvement in the treated condition. A preferred dosing frequency is one-two times per week. The dosing periods, e.g., two weeks, may be interrupted by a wash-out period, typically of 1-4 weeks. The treatment, e.g., involving repeating dosing and wash-out periods, may continue over an extended period of several months or more.

The amount of composition administered at each dose is preferably between about 1 to 50 mg vesicle lipid per kg of body weight. In a preferred embodiment, the composition is administered one time per week, at a dose of about 5-20 mg lipid/kg body weight. A typical dose for an 80 kg individual would be between about 0.4 to 1.6 grams lipid, corresponding to between 2-8 ml of a particle suspension containing 200 ms lipid/ml. Administration may be by intravenous (IV) injection, or IV drip (infusion). The particle composition may be suspended in sterile saline or in a nutritional or drug-containing buffer or medium, such as a glucose/salt medium, to combine composition treatment with other parenteral therapy.

Treatment of a number of disease conditions by intravenous infusion of liposome SUVs has been described above. These diseases may also be treated by intravenous administration of serum particles like those formed with IV administration of such liposomes, as described above. Disease treatment with lipoprotein particle composition differs from treatment with liposome SUVs in a few aspects. Because apoprotein particles can be expected to form *in vivo* only after a several hour period, a substantial percentage of administered liposome SUVs are cleared before effective apolipoprotein particle formation occurs. Thus, substantially less liposome lipid is required in the use of the lipoprotein particle composition, where the particles are active immediately on administration.

In addition, activity of the lipoprotein particles formed *in vivo* is limited by the amount of serum apoprotein available for binding to the administered liposomes. The lipoprotein particle composition can be produced with relatively high concentrations of apoproteins, permitting therapeutic effectiveness at significantly lower amounts of administered liposomal lipid.

Finally, the lipoprotein particle composition does not rely on depleting existing serum apoproteins A and C, either in free or HDL form, from the bloodstream; thus, administration of the lipoprotein particle composition tends to augment, rather than replace, native HDL particles.

### A. Treatment of Acute Renal Failure

In one embodiment, this method is employed for use in treating acute renal failure (ARF) in a subject, as evidenced by elevated levels of serum creatinine. ARF is typically detected by determination of glomerular filtration rate (GFR) or blood urea nitrogen or serum creatinine levels. GFR is the rate of ultrafiltration of plasma across the walls of the glomerular capillaries and measurement of total GFR of both kidneys provides a sensitive index of overall renal excretory function. Normal renal excretory function is indicated by a GFR of about 125 Ml/min (180 L/day), although when renal excretory capacity is impaired, total GFR declines.

Often, measurements of urea and creatinine concentrations are used to assess the glomerular filtration rate. Both substances are produced at a relatively constant rate by the liver and muscles; an increase in their respective serum concentrations occurs as GFR declines due to the fact that both compounds undergo complete glomerular filtration and are not reabsorbed by the renal tubules. Creatinine provides a more reliable index of GFR than urea because urea can back diffuse more completely from tubule lumen to peritubular blood than creatinine.

Chemical analysis of both urine and serum samples are useful indicators of ARF. For example, the range of urine osmolalities that can be achieved by an individual with normal-functioning kidneys (40 to 1200 mosmol/kg) is much larger than the range achievable in diseased kidneys (250-350 mosmol/kg). Typically, acute renal failure is characterized by urine osmolalities of below about 400 mosmol/kg, urine sodium concentrations above about 40 mmol/L, a ratio of urine-to-plasma creatinine levels below 20, and a fractional excretion of filtered sodium, defined as the ratio of urine sodium concentration/serum sodium concentration to urine creatinine concentration/serum creatinine concentration multiplied by 100, of about 2 (Harrison 1991). In this embodiment, a person having acute renal failure, as evidenced by a ratio of urine-to-plasma creatinine levels less than about 20, is treated by IV administration of the lipoprotein composition of the invention, as described above. The therapeutic effect of the treatment is monitored by assaying urine-to-serum creatinine levels (or other characteristic of ARF discussed above). Treatment is continued, i.e., with repeated administration 1-2 times/week, until a significant improvement (rise) in urine-to-serum creatinine level is achieved.

### B. Treatment of Hypertension

In another embodiment, this method is employed for the treatment of hypertension. Hypertension refers to elevated arterial pressure, and is typically reported as a ratio of systolic pressure (arterial pressure during contraction of the heart muscle) to diastolic pressure (residual arterial pressure during relaxation of the heart muscle), reported in units of mm Hg. A normal diastolic blood pressure is between about 60-85 mm Hg. Diastolic pressures above 85 mm Hg are generally diagnostic of hypertension.

In this embodiment, a subject having elevated blood pressure, i.e., diastolic pressure above about 85 mm Hg, is treated by IV administration of the lipoprotein particle composition. Therapeutic effectiveness is followed by monitoring blood pressure, preferably diastolic blood pressure. Treatment is continued, e.g., by repeated administration of the composition 1-2 times/week, until a significant reduction, and preferably at least a 10% reduction in diastolic blood pressure is observed.

### C. Treatment of Diseases Associated with Elevated Lp(a)

In another embodiment, this method is used to reduce the serum Lp(a) concentration in a person at risk for developing a disease condition associated with a chronic elevated serum Lp(a) concentration. Conditions associated with elevated Lp(a) concentrations include, for example, gout, breast cancer and hyperthyroidism, as described previously.

In this general embodiment, a subject having elevated Lp(a) level, i.e., a serum Lp(a) level above 25 mg/dl, Hg, is treated by IV administration of the lipoprotein particles of the invention. Therapeutic effectiveness is followed by monitoring serum Lp(a) level, and treatment is continued, by repeated administration of the composition 1-2 times/week, until a significant reduction, preferably at least a 20-40% reduction, in serum Lp(a) level is observed.

More specific embodiments include methods of treating gout, breast cancer or hyperthyroidism in a subject having one of these conditions and an elevated serum Lp(a) concentration. Studies have shown that serum Lp(a) concentrations are elevated in many subjects with gout (Takahashi, 1995), various types of cancer, such as breast cancer (Kokoglu, 1994), and hyperthyroidism (Yamamoto, 1995). The purpose of this method is to treat the clinical disease by lowering Lp(a) levels, as one of the underlying factors contributing to the disease.

Treatment, in accordance with this embodiment, involves first determining serum Lp(a) concentration in a person having one of the above clinical conditions. A patient having an elevated Lp(a) level is then selected as a candidate for the composition treatment method, as described above, typically as an adjunct to another treatment method, such as surgery, chemotherapy, or radiation therapy in the case of breast cancer. Treatment is maintained until a significant reduction in Lp(a) is observed and preferably throughout the treatment period for the clinical disease.

### C. Treatment of Restenosis

Restenosis has been described above. This embodiment relates to a method of reducing the extent of restenosis following procedures such as balloon angioplasty or surgical resectioning of vascular tissue. Typically, in this method, a person undergoing such a procedure that can lead to restenosis is given one or more pretreatment administrations of the lipoprotein particle composition, particularly where existing Lp(a) levels are elevated, to achieve a reduction in such levels. Following the procedure, the patient is again monitored for Lp(a) serum concentrations, and given further particle injections if necessary to maintain or achieve low Lp(a) levels, e.g., 20 mg/dl or lower. The treatment may be discontinued after a period of several weeks or more when the risk of restenosis has passed.

The following examples illustrate the present invention.

### MATERIALS AND METHODS

Egg phosphatidylcholine (egg PC) recovered from egg yolk may be prepared according to known methods (Shinitsky, *et al.*, 1974). High purity egg PC may also be purchased from Avanti Polar Lipids (Alabaster, AL), Lipoid KG (Ludwigshafen, Germany), or from Sigma (St. Louis, MO). The egg PC used to form small unilamellar vesicles as described below was determined to be greater than 99% pure by thin layer chromatography (TLC) analysis.

### EXAMPLE 1

### PREPARATION OF SMALL UNILAMELLAR VESICLES: SONICATION

Egg PC dissolved in chloroform was placed in a 100 ml vessel and dried to a thin film under an inert atmosphere of nitrogen. Sterile saline was added to the lipid film to a final concentration of about 100 mg/ml, and the lipid film was hydrated with swirling. The resulting multilamellar vesicle (MLV) suspension was then bath sonicated for 1 hour using a Heat System Sonicator, Model 375W, at a power setting of 40-50% full value. The temperature of the suspension was maintained at about 4°C during sonication. Large vesicles or MLVs were separated from the sonicated suspension by ultracentrifugation at 100,000 g for 1 hour (Barenholz, 1977). The remaining suspension of SUVs, having a concentration of about 100 mg/ml, was then filter sterilized.

### EXAMPLE 2

### PREPARATION OF SMALL UNILAMELLAR VESICLES: EXTRUSION

Homogeneous small unilamellar vesicles of egg PC with an average diameter of 39 ± 8 nm, in 0.15 M NaCl were prepared by extrusion using serial filtration through polycarbonate filters in a GH 76-400 pressure cell (Nucleopore) (Anselem, *et al.*, 1993). Liposomal size was determined using a Coulter model N4 sub-micron particle analyzer equipped with a size distribution processor analyzer (Barenholz, *et al.*, 1993). The final extrusion step was through a 0.05 micrometer pore polycarbonate filter. Egg PC SUVs were sterilized by filtration through sterile 0.22 micrometer Millipore filters.

### EXAMPLE 3

### EFFECTS OF EGG SUV PC TREATMENT ON THREE MALE SUBJECTS

Suspensions of small unilamellar vesicles prepared as described in Examples 1 and 2 above were administered over a 7 week period to three subjects.

**Table 2**

| **Subject No.** | **Gender** | **Age** |
|---|---|---|
| (1) | male | 40 |
| (2) | male | 54 |
| (3) | male | 64 |

The following treatments were administered to each of the three subjects:

**Table 3**

| **Treatment No.** | **Week** | **Treatment Regime** |
|---|---|---|
| 1 | 1 | IV infusion of 250 ml of 0.9% NaCl solution, followed by IV infusion of 200 mg SUVs/kg body weight |
| 2 | 2 | IV infusion of 300 mg SUVs/kg body weight |
| 3 | 3 | IV infusion of 300 mg SUVs/kg body weight |
| 4 | 4 | IV infusion of 300 mg SUVs/kg body weight |
| - | 5-8 | IV administration suspended |
| 5 | 9 | treatment resumed with IV infusion of 300 mg SUVs/kg body weight |
| 6 | 10 | IV infusion of 300 mg SUVs/kg body weight |
| 7 | 11 | IV infusion of 300 mg/kg SUV/kg body weight |

Over the course of treatment, pulse rate, blood pressure, body temperature and body weight were monitored and no significant changes were observed. None of the subjects reported hypersensitivity reaction or other adverse effects relating to SUV treatment.

Subject No. (1) reported an improvement in skin texture, noting that his skin appeared to be more "silky". Subject No. (3) reported an improvement in his ability to perform strenuous physical activity and in the condition of his gums.

Laboratory test results for each of the subjects revealed no abnormalities in liver function, renal function, glucose, electrolytes, CPK and aldolase levels. Complete blood count, coagulation tests and blood hormone levels (*e.g.*, thyroid stimulating hormone (TSH), cortisol and testosterone) were within normal ranges and did not change significantly over the course of treatment. EKG traces for each of the subjects were normal over the course of treatment and abdominal ultrasound performed before and after completion of the study were normal and revealed no indications of fatty liver.

### EXAMPLE 4

### OSMOTIC FRAGILITY OF RED BLOOD CELLS

The osmotic fragility of red blood cells from each of subjects (1)-(3) was determined over the period of SUV treatment. Hypotonic solutions of NaCl at pH 7.4 were used to determine the concentration at which cell lysis occurred for both fresh red blood cells and for red blood cells which were incubated for 24 hours at 37° C. The concentration of NaCl at which lysis occurred in 50% of the cells (*e.g.*, the median corpuscular fragility or MCF) was used as an indication of cell osmotic fragility.

Red blood cell samples (both pre and post infusion) exhibited MCF values at 20°C and pH 7.4 within a concentration range of 4.0-4.5 g NaCl/ml for fresh red blood cells and between 4.65-5.9 g NaCl/ml for red blood cells incubated at pH 7.4 at a temperature of 37° C for 24 hours.

The MCF values for all samples remained in the normal range over the course of the study.

### EXAMPLE 5

### RATE OF APPEARANCE AND CLEARANCE OF SUVs IN THE CIRCULATION

Serum phospholipid levels were determined in order to follow the rate of appearance and clearance of intravenously administered liposome in the circulation. Serum isolated from blood samples taken from each of the subjects at various times after SUV infusion were extracted by standard methods. PC concentrations in blood following liposome injection were determined by measuring the increase in inorganic phosphate (Pᵢ) in serum; this method of measuring PC was confirmed by an enzymatic assay specific for choline phospholipids.

PC levels in serum reached maximal levels at the end of the infusion period, decreased considerably over the next 24 hours following infusion and gradually increased to preinfusion values at day 8. Based upon this finding, the treatment regime described in Table 2 (*e.g.*, infusion every 7 days) was followed.

### EXAMPLE 6

### ISOLATION AND CHARACTERIZATION OF LIPOPROTEIN PARTICLES

Liposomes were recovered from blood samples withdrawn from the patients at various times after infusion and isolated by centrifugation at a density of 1.006 g/ml or 1.019 g/ml. The recovered post-infusion liposomes were characterized as a distinct lipoprotein fraction just above LDL. The post-infusion liposomes in the isolated lipoprotein fraction were then examined by electron microscopy and observed to form bilayers. Electron micrographs of negatively stained LDL and VLDL fractions revealed that both the LDL and VLDL fractions also contained liposomes.

The composition of the recovered lipoprotein fraction was determined by SDS-PAGE on acrylamide gel. Based upon electrophoretic mobility, the isolated lipoprotein fraction was determined to contain Apo A-1, Apo E, and Apo Cs. Apo B was not detected. The recovered lipoprotein fraction was also found to contain free cholesterol. The composition and characteristics of the recovered lipoprotein fraction are summarized in Table 4 below.

**Table 4**

| **New Lipoprotein Fraction** | |
|---|---|
| **Density (g/ml)** | **between 1.006-1.019** |
| Electron microscopy | bilayers **size estimate?** |
| Apoproteins: | Apo A-1 |
| | Apo Cs |
| | Apo E |
| Lipids | phospholipid |
| | free cholesterol |

### EXAMPLE 7

### POST SUV TREATMENT: PLASMA ANALYSIS

Plasma isolated from blood samples taken from each of the subjects at various times after SUV infusion was analyzed for free cholesterol content, levels of LDL-C, HDL-C, Apo A-1, triglycerides, and Lp(a). Twenty four hours after infusion with SUVs, red blood cell free cholesterol levels were found to be reduced by 27%, 10.5% and 18% in subjects No. (1), (2), and (3), respectively, and returned to baseline values 24 hours later. The change in red blood cell cholesterol content following SUV infusion did not affect red blood cell osmotic fragility. No signs of homolysis were observed, as indicated by constant levels of serum haptoglobin and free hemoglobin observed both before and after SUV treatment. Post SUV infusion serum samples were centrifuged at a density of 1.019 g/ml to isolate the LDL-C fraction. No significant change in the post-infusion LDL-C fraction was observed.

Plasma analysis also revealed that during the course of treatment, profiles of HDL-C and apo A-1 levels tended to rise. Triglyceride levels remained substantially unchanged. Lp(a) levels were found to be substantially reduced. Levels of free cholesterol in plasma were found to increase after SUV infusion, and returned to baseline levels as the liposomes were cleared through the circulation.

**Table 5**

| **Post SUV Treatment: Plasma Analysis Summary** | | | | | |
|---|---|---|---|---|---|
| **Free cholesterol** | **LDL-C** | **HDL-C** | **Apo A-1** | **Triglycerides** | **Lp(a)** |
| increased | no change | increased | increased | no change | reduced |

## Claims

1. Use of a suspension of small unilamellar liposomes composed predominantly of phosphatidylcholine phospholipids having phase transition temperatures in the range between about -10 and 37°C for the preparation of a medicine for reducing the serum Lp(a) concentration in a subject at risk for developing a disease condition associated with chronic, elevated serum Lp(a) concentrations.

2. Use of a suspension according to claim 1 for the preparation of a medicine for treating gout by reducing a chronic, elevated serum Lp(a) concentration in a subject suffering from gout.

3. Use of a suspension according to claim 1 for the preparation of a medicine for treating breast cancer by reducing a chronic, elevated serum Lp(a) concentration in a subject suffering from breast cancer.

4. Use of a suspension according to claim 1 for the preparation of a medicine for treating hyperthyroidism by reducing a chronic, elevated serum Lp(a) concentration in a subject suffering from hyperthyroidism.

5. Use of a suspension according to claim 1 for the preparation of a medicine for inhibiting restenosis in a subject following percutaneaous transluminal coronary angioplasty or surgical resection of vascular tissue.

6. Use of a suspension according to claim 1 for the preparation of a medicine for treating periodontal disease.

7. Use according to claim 1 wherein said suspension is for intravenously administering to the subject, and wherein said administering is to be repeated over a period of at least two weeks, and in an amount effective to produce a significant reduction in serum Lp(a) concentration.

8. Use according to claim 1, wherein said administering includes administering liposomes having sizes ranging between 0,02 and 0,12 µm.

9. Use according to claim 1, wherein said administering includes administering liposomes composed of egg phosphatidylcholine.

10. Use according to claim 1, wherein said administering includes administering liposomes composed of phospholipid having a phase transition temperature of less than about 5°C.

11. Use according to claim 1, wherein said repeating includes administration of said liposome suspension at least one time per week and at a dose of between about 50 - 1000 mg lipid/kg body weight.

12. Use according to claim 1, wherein said medicine is for treating gout, breast cancer or hyperthyroidism in a subject having an elevated serum Lp(a) concentration.

13. Use according to claim 1, wherein said medicine is for inhibiting restenosis in a subject following percutaneous transluminal coronary angioplasty or surgical resection of vascular tissue.

14. Use according to claim 13, in which the subject is determined to have an elevated serum Lp(a) concentration.

15. A therapeutic lipoprotein composition for intravenous administration to a subject, obtainable by mixing small unilamellar vesicles composed of phosphatidylcholine phospholipids having phase transition temperatures between about
- 10 and 37°C with a mixture of apoproteins consisting essentially of apoproteins from classes A and C, under conditions effective to form lipoprotein particles composed of said vesicles and associated A and C apoproteins.

16. A composition according to claim 15, wherein said mixing includes intravenously administering said vesicles to a human subject and allowing the administered vesicles to circulate in the bloodstream for a period of at least 2 hours, and which further includes removing a blood sample from the subject, and isolating from serum obtained from the blood sample, lipoprotein particles having a density between about 1,0006 and 1,019 g/ml.

17. A lipoprotein particle composition consisting essentially of phosphatidylcholine phospholipids having phase transition temperatures between about -10 and 37°C with a mixture of apoproteins consisting essentially of apoproteins from classes A and C.

18. The composition of claims 15 and 17, wherein the vesicles are empty vesicles.

19. The composition of claims 15, 16 and 17, wherein the vesicles have sizes between 0,03 and 0,08 µm.

20. The composition of claims 15, 16 and 17, wherein the vesicles are composed of egg phosphatidylcholine.

21. The composition of claims 15, 16 and 17, wherein the apoproteins include apo a-1 and class C apoproteins.

22. The composition of claim 21, wherein the apoproteins are human recombinant apoproteins.

23. The composition of claims 15 and 17, wherein the lipoprotein particles are **characterized by** a density between about 1,006 and 1,019 g/ml.

24. The composition of claim 16, wherein the lipoprotein particles are further treated to remove associated apoprotein apo E.

25. Use of a lipoprotein composition according to claim 15 for the preparation of a medicine for treating acute renal failure in a subject, as evidenced by a ratio of urine-to-plasma creatinine levels less than about 20.

26. Use of a lipoprotein composition according to claim 15 for the preparation of a medicine for treating hypertension in a subject having elevated blood pressure.

27. Use of the therapeutic lipoprotein composition of claim 15 for the preparation of a medicine for treating a disease state which is associated with elevated levels of Lp(a),
wherein said lipoprotein composition is for administering to a subject having an elevated serum Lp(a) level in a therapeutically effective dose, wherein said administering is to be repeated, if necessary, until a measurable reduction in serum Lp(a) level is observed.

28. Use of the therapeutic lipoprotein composition of claim 15 for the preparation of a medicine for treating a disease state which is responsive to intravenous administration of doses of small unilamellar phosphatidylcholine vesicles, in the dosage range between about 50-1000 mg vesicle lipid/kg subject body weight, wherein said lipoprotein composition is for administering to a subject in need of such treatment in a therapeutically effective dose, wherein said administering is to be repeated, if necessary, until a measurable improvement in said disease state is observed.

29. Use according to claim 28, wherein said medicine is for treating acute renal failure in a subject, as evidenced by a ratio of urine-to-plasma creatinine levels less than about 20, wherein said administering is to be continued until a significant increase in the urine-to-plasma creatinine ratio is observed.

30. Use according to claim 28, wherein said medicine is for treating hypertension in a subject having elevated blood pressure, wherein said administering is to be continued until a significant reduction in diastolic blood pressure is achieved.

## Patentansprüche

1. Verwendung einer Suspension von kleinen unilamellaren Liposomen, welche hauptsächlich aus Phosphatidylcholin-Phospholipiden mit Phasenübergangstemperaturen in dem Bereich zwischen etwa -10 und 37 °C zusammengesetzt sind, zur Herstellung einer Medizin zur Reduzierung der Serum Lp(a) Konzentration in einem Subjekt, welches einem Risiko für eine Entwicklung eines mit chronisch erhöhten Serum Lp(a) Konzentrationen verbundenen Krankheitszustands ausgesetzt ist.

2. Verwendung einer Suspension nach Anspruch 1 zur Herstellung einer Medizin zur Behandlung von Gicht durch Reduktion einer chronisch erhöhten Serum Lp(a) Konzentration in einem an Gicht leidenden Subjekt.

3. Verwendung einer Suspension nach Anspruch 1 zur Herstellung einer Medizin zur Behandlung von Brustkrebs durch Reduzierung einer chronisch erhöhten Serum Lp(a) Konzentration in einem an Brustkrebs leidenden Subjekt.

4. Verwendung einer Suspension nach Anspruch 1 zur Herstellung einer Medizin zur Behandlung von Hyperthyroidismus durch Reduzierung einer chronisch erhöhten Serum Lp(a) Konzentration in einem an Hyperthyroidismus leidenden Subjekt.

5. Verwendung einer Suspension nach Anspruch 1 zur Herstellung einer Medizin zur Inhibierung von Restenose in einem Subjekt nach perkutaner transluminaler koronarer Angioplastie oder operativer Resektion von Gefäßgewebe.

6. Verwendung einer Suspension nach Anspruch 1 zur Herstellung einer Medizin zur Behandlung einer Paradontalerkrankung.

7. Verwendung nach Anspruch 1, **dadurch gekennzeichnet, dass** die Suspension zur intravenösen Verabreichung an das Subjekt ist, und wobei die Verabreichung über einen Zeitraum von mindestens zwei Wochen wiederholt werden soll, in einer Menge, die effektiv zur Bewirkung einer signifikanten Reduktion der Serum Lp(a) Konzentration ist.

8. Verwendung nach Anspruch 1, **dadurch gekennzeichnet, dass** die Verabreichung es einschließt, Liposomen mit Größen im Bereich zwischen 0,02 und 0,12 µm zu verabreichen.

9. Verwendung nach Anspruch 1, **dadurch gekennzeichnet, dass** die Verabreichung es einschließt, Liposomen zu verabreichen, welche aus Ei-Phosphatidylcholin zusammengesetzt sind.

10. Verwendung nach Anspruch 1, **dadurch gekennzeichnet, dass** das Verabreichen es einschließt, Liposomen zu verabreichen, die aus Phospholipid mit einer Phasenübergangstemperatur von weniger als etwa 5° C zusammengesetzt sind.

11. Verwendung nach Anspruch 1, **dadurch gekennzeichnet, dass** das Wiederholen eine Verabreichung der Liposomensuspension mindestens einmal pro Woche und in einer Dosis von zwischen etwa 50 - 1000 mg Lipid / kg Körpergewicht einschließt.

12. Verwendung nach Anspruch 1, **dadurch gekennzeichnet, dass** die Medizin zur Behandlung von Gicht, Brustkrebs oder Hyperthyroidismus in einem Subjekt mit einer erhöhten Serum Lp(a) Konzentration ist.

13. Verwendung nach Anspruch 1, **dadurch gekennzeichnet, dass** die Medizin zur Inhibierung von Restenose in einem Subjekt nach perkutaner transluminaler koronarer Angioplastie oder operativer Resektion von Gefäßgewebe ist.

14. Verwendung nach Anspruch 13, **dadurch gekennzeichnet, dass** bestimmt wird, dass das Subjekt eine erhöhte Serum Lp(a) Konzentration aufweist.

15. Eine therapeutische Lipoproteinzusammensetzung zur intravenösen Verabreichung an ein Subjekt, erhältlich durch das Mischen von kleinen unilamellaren Vesikeln, zusammengesetzt aus Phospatidylcholin-Phospholipiden mit Phasenübergangstemperaturen zwischen etwa -10 und 37 °C, mit einer Mischung von Apoproteinen, welche im Wesentlichen aus Apoproteinen der Klassen A und C bestehen, unter Bedingungen, die effektiv zur Bildung von aus den Vesikeln und assoziierten A und C Apoproteinen zusammengesetzten Lipoproteinpartikeln sind.

16. Eine Zusammensetzung nach Anspruch 15, **dadurch gekennzeichnet, dass** das Mischen es einschließt, die Vesikel intravenös einem menschlichen Subjekt zu verabreichen und es den verabreichten Vesikeln zu erlauben, für einen Zeitraum von mindestens zwei Stunden in dem Blutstrom zu zirkulieren, und welches es ferner einschließt, eine Blutprobe des Subjekts zu entnehmen, und aus aus der Blutprobe enthaltenem Serum Lipoproteinpartikel mit einer Dichte von zwischen 1,006 und 1,019 g/ml zu isolieren.

17. Eine Zusammensetzung von Lipoproteinpartikeln, im Wesentlichen bestehend aus Phosphatidylcholin-Phospholipiden mit Phasenübergangstemperaturen zwischen etwa -10 und 37 °C mit einer Mischung von Apoproteinen, welche im Wesentlichen aus Apoproteinen der Klassen A und C besteht.

18. Die Zusammensetzung nach den Ansprüchen 15 und 17,
**dadurch gekennzeichnet, dass** die Vesikel leere Vesikel sind.

19. Die Zusammensetzung nach den Ansprüchen 15, 16 und 17, **dadurch gekennzeichnet, dass** die Vesikel Größen zwischen 0,03 und 0,08 µm aufweisen.

20. Die Zusammensetzung nach den Ansprüchen 15, 16 und 17, **dadurch gekennzeichnet, dass** die Vesikel aus Ei-Phosphatidylcholin aufgebaut sind.

21. Die Zusammensetzung nach den Ansprüchen 15, 16 und 17, **dadurch gekennzeichnet, dass** die Apoproteine Apo a-1 und Klasse C Apoproteine einschließen.

22. Die Zusammensetzung nach Anspruch 21, **dadurch gekennzeichnet, dass** die Apoproteine humanere rekombinante Apoproteine sind.

23. Die Zusammensetzung nach den Ansprüchen 15 und 17, **dadurch gekennzeichnet, dass** die Lipoproteinpartikel durch eine Dichte zwischen etwa 1,006 und 1,019 g/ml charakterisiert sind.

24. Die Zusammensetzung nach Anspruch 16, **dadurch gekennzeichnet, dass** die Lipoproteinpartikel weiter behandelt werden, um assoziiertes Apoprotein Apo E zu entfernen.

25. Verwendung einer Lipoproteinzusammensetzung nach Anspruch 15 zur Herstellung einer Medizin zur Behandlung von akutem Nierenversagen in einem Subjekt, belegt durch ein Verhältnis von Urin-zu-Plasma Creatinin-Spiegel von weniger als etwa 20.

26. Verwendung einer Lipoproteinzusammensetzung nach Anspruch 15 zur Herstellung einer Medizin zur Behandlung von Bluthochdruck in einem Subjekt mit erhöhtem Blutdruck.

27. Verwendung der therapeutischen Lipoproteinzusammensetzung nach Anspruch 15 zur Herstellung einer Medizin zur Behandlung eines Erkrankungszustandes, welcher mit erhöhten Spiegeln von Lp(a) assoziiert ist, wobei die Lipoproteinzusammensetzung zur Verabreichung an ein Subjekt ist, welches einen erhöhten Serum Lp(a) Spiegel aufweist, in einer therapeutisch effizienten Dosis, wobei die Verabreichung wenn nötig zu wiederholen ist, bis eine messbare Reduktion des Serum Lp(a) Spiegels beobachtet wird.

28. Verwendung der therapeutischen Lipoproteinzusammensetzung nach Anspruch 15 zur Herstellung einer Medizin zur Behandlung eines Erkrankungszustands, welcher auf intravenöse Verabreichung von Dosen von kleinen unilamellaren Phosphatidylcholin Vesikeln in dem Dosisbereich zwischen etwa 50 - 1000 mg Vesikel-Lipid / kg Körpergewicht des Subjekts reagiert, wobei die Lipoproteinzusammensetzung zur Verabreichung an ein Subjekt mit Bedarf für eine solche Behandlung in einer therapeutisch effizienten Dosis ist, wobei die Verabreichung wenn nötig zu wiederholen ist, bis eine messbare Verbesserung des Erkrankungszustands beobachtet wird.

29. Verwendung nach Anspruch 28, **dadurch gekennzeichnet, dass** die Medizin zur Behandlung von akutem Nierenversagen in einem Subjekt ist, wie es durch ein Verhältnis von Urin-zu-Plasma Creatinin-Spiegel von weniger als etwa 20 belegt ist, wobei die Verabreichung fortgesetzt werden soll, bis eine signifikante Steigerung in dem Urin-zu-Plasma Creatinin-Verhältnis beobachtet wird.

30. Verwendung nach Anspruch 28, **dadurch gekennzeichnet, dass** die Medizin zur Behandlung von Bluthochdruck in einem Subjekt mit erhöhtem Blutdruck ist, wobei die Verabreichung fortgeführt werden soll, bis eine signifikante Reduktion des diastolischen Blutdrucks erreicht wird.

## Revendications

1. Utilisation d'une suspension de petits liposomes unilamellaires composés principalement de phospholipides phosphatidylcholine ayant des températures de transition de phase dans le domaine compris entre environ -10°C et 37°C pour la préparation d'un médicament pour réduire la concentration sérique de Lp(a) chez un sujet ayant un risque de développer un état pathologique associé à des concentrations sériques élevées et chroniques de Lp(a).

2. Utilisation d'une suspension selon la revendication 1 pour la préparation d'un médicament pour le traitement de la goutte en réduisant une concentration sérique élevée et chronique de Lp(a) chez un sujet souffrant de la goutte.

3. Utilisation d'une suspension selon la revendication 1 pour la préparation d'un médicament pour le traitement du cancer du sein en réduisant une concentration sérique élevée et chronique de Lp(a) chez un sujet souffrant du cancer du sein.

4. Utilisation d'une suspension selon la revendication 1 pour la préparation d'un médicament pour le traitement de l'hyperthyroïdie en réduisant une concentration sérique élevée et chronique de Lp(a) chez un sujet souffrant d'hyperthyroïdie.

5. Utilisation d'une suspension selon la revendication 1 pour la préparation d'un médicament pour inhiber la resténose chez un sujet à la suite d'une angioplastie coronarienne transluminale percutanée ou d'une résection chirurgicale de tissus vasculaires.

6. Utilisation d'une suspension selon la revendication 1 pour la préparation d'un médicament pour le traitement d'une affection parodontale.

7. Utilisation selon la revendication 1, où ladite suspension est destinée à une administration par voie intraveineuse au sujet, et où ladite administration doit être répétée sur une période d'au moins deux semaines, et en une quantité efficace pour produire une réduction significative de la concentration sérique de Lp(a).

8. Utilisation selon la revendication 1, où ladite administration comprend l'administration de liposomes ayant une taille dans le domaine compris entre 0,02 µm et 0,12 µm.

9. Utilisation selon la revendication 1, où ladite administration comprend l'administration de liposomes composés de phosphatidylcholine d'oeuf.

10. Utilisation selon la revendication 1, où ladite administration comprend l'administration de liposomes composés de phospholipides ayant une température de transition de phase inférieure à environ 5°C.

11. Utilisation selon la revendication 1, où ladite répétition comprend l'administration de ladite suspension de liposomes au moins une fois par semaine et à une dose comprise entre environ 50 et 1000 mg de lipides/kg de poids corporel.

12. Utilisation selon la revendication 1, où ledit médicament est destiné au traitement de la goutte, du cancer du sein ou de l'hyperthyroïdie chez un sujet ayant une concentration sérique élevée de Lp(a).

13. Utilisation selon la revendication 1, où ledit médicament est destiné à inhiber la resténose chez un sujet à la suite d'une angioplastie coronarienne transluminale percutanée ou d'une résection chirurgicale de tissus vasculaires.

14. Utilisation selon la revendication 13, dans laquelle le sujet est considéré comme ayant une concentration sérique élevée de Lp(a).

15. Composition de lipoprotéines thérapeutique destinée à l'administration intraveineuse à un sujet, pouvant être obtenue en mélangeant de petites vésicules unilamellaires composées de phospholipides phosphatidylcholine ayant des températures de transition de phase comprises entre environ -10°C et 37°C avec un mélange d'apoprotéines constitué essentiellement d'apoprotéines de classe A et C, dans des conditions efficaces pour former des particules lipoprotéiques composées desdites vésicules et apoprotéines A et C associées.

16. Composition selon la revendication 15, où ledit mélange consiste à administrer par voie intraveineuse lesdites vésicules à un sujet humain et à laisser les vésicules administrées circuler dans la circulation sanguine pendant une période d'au moins 2 heures, et qui consiste en outre à prélever un échantillon de sang chez le sujet, et à isoler du sérum obtenu de l'échantillon de sang, les particules lipoprotéiques ayant une masse volumique comprise entre environ 1,0006 et 1,019 g/ml.

17. Composition de particules lipoprotéiques constitué essentiellement de phospholipides phosphatidylcholine ayant des températures de transition de phase comprises entre environ -10°C et 37°C avec un mélange d'apoprotéines constitué essentiellement d'apoprotéines de classe A et C.

18. Composition selon les revendications 15 et 17, où les vésicules sont des vésicules vides.

19. Composition selon les revendications 15, 16 et 17, où les vésicules ont des tailles entre 0,03 µm et 0,08 µm.

20. Composition selon les revendications 15, 16 et 17, où les vésicules sont composées de phosphatidylcholine d'oeuf.

21. Composition selon les revendications 15, 16 et 17, où les apoprotéines comprennent des apoprotéines apo a-1 et de classe C.

22. Composition selon la revendication 21, où les apoprotéines sont des apoprotéines recombinantes humaines.

23. Composition selon les revendications 15 et 17, où les particules lipoprotéiques sont **caractérisées par** une densité entre environ 1,006 et 1,019 g/ml.

24. Composition selon la revendication 16, où les particules lipoprotéiques sont traitées en outre pour éliminer l'apoprotéine associée apo E.

25. Utilisation d'une composition de lipoprotéines selon la revendication 15 pour la préparation d'un médicament pour le traitement de l'insuffisance rénale aiguë chez un sujet, mise en évidence par un rapport des niveaux de créatinine dans l'urine par rapport au plasma inférieurs à environ 20.

26. Utilisation d'une composition de lipoprotéines selon la revendication 15 pour la préparation d'un médicament pour le traitement de l'hypertension chez un sujet ayant une pression artérielle élevée.

27. Utilisation de la composition de lipoprotéines thérapeutique selon la revendication 15 pour la préparation d'un médicament pour le traitement d'un état pathologique qui est associé à des niveaux élevés de Lp(a), où ladite composition de lipoprotéines doit être administrée à un sujet ayant un niveau sérique élevé de Lp(a) en une dose thérapeutiquement efficace, où ladite administration doit être répétée, si nécessaire, jusqu'à ce qu'une réduction mesurable du niveau sérique de Lp(a) soit observée.

28. Utilisation de la composition de lipoprotéines thérapeutique selon la revendication 15 pour la préparation d'un médicament pour le traitement d'un état pathologique qui est sensible à l'administration par voie intraveineuse de doses de petites vésicules unilamellaires de phosphatidylcholine, dans le domaine de dosage entre environ 50 à 1000 mg de vésicules lipidiques/kg de poids corporel du sujet, où ladite composition de lipoprotéines doit être administrée chez un sujet ayant besoin d'un tel traitement en une dose thérapeutiquement efficace, où ladite administration doit être répétée, si nécessaire, jusqu'à ce qu'une amélioration mesurable dudit état pathologique soit observée.

29. Utilisation selon la revendication 28, où ledit médicament est destiné au traitement de l'insuffisance rénale aiguë chez un sujet, mise en évidence par un rapport des niveaux de créatinine dans l'urine par rapport au plasma inférieurs à environ 20, où ladite administration doit être poursuivie jusqu'à ce qu'une augmentation significative du rapport des niveaux de créatinine de l'urine par rapport au plasma soit observée.

30. Utilisation selon la revendication 28, où ledit médicament est destiné au traitement de l'hypertension chez un sujet ayant une pression artérielle élevée, où ladite administration doit être poursuivie jusqu'à ce qu'une réduction significative de la pression sanguine diastolique soit atteinte.
